Europäisches Patentamt

European Patent Office

Office européen des brevets

(19)

(11) Veröffentlichungsnummer : **0 116 363**
**B1**

(12) **EUROPÄISCHE PATENTSCHRIFT**

(45) Veröffentlichungstag der Patentschrift :
04.03.87

(51) Int. Cl.⁴ : **A 61 F   5/441**

(21) Anmeldenummer : **84101213.1**

(22) Anmeldetag : **07.02.84**

(54) **Kolostomiebeutel mit einer Einrichtung zur Filterung und kontrollierten Abführung der Gase.**

(30) Priorität : 09.02.83 DE 3304312

(43) Veröffentlichungstag der Anmeldung :
22.08.84 Patentblatt 84/34

(45) Bekanntmachung des Hinweises auf die Patenterteilung : 04.03.87 Patentblatt 87/10

(84) Benannte Vertragsstaaten :
CH FR GB IT LI NL SE

(56) Entgegenhaltungen :
EP-A- 0 037 944
EP-A- 0 064 044
DE-A- 2 550 766
DE-A- 2 928 274
DE-C-   236 255
DE-U- 8 133 695
GB-A-   576 181
GB-A- 1 295 252
GB-A- 2 029 764
GB-A- 2 059 797
GB-A- 2 094 153
US-A- 2 054 535
US-A- 3 055 368
US-A- 3 865 109
US-A- 4 232 672
US-A- 4 367 742

(73) Patentinhaber : **HELSA-WERKE Helmut Sandler
GmbH & Co. KG
Bayreuther Strasse 3-4
D-8586 Gefrees (DE)**

(72) Erfinder : **Rupprecht, Michael, Dipl.-Ing. (FH)
Albert-Schweitzer-Strasse 11
D-8586 Gefrees (DE)**

(74) Vertreter : **LOUIS, PÖHLAU, LOHRENTZ & SEGETH
Kesslerplatz 1 P.B. 3055
D-8500 Nürnberg (DE)**

EP 0 116 363 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft einen Kolostomiebeutel mit einer Einrichtung zur Filterung und kontrollierten Abführung übelriechender Darmgase, der eine besondere Filterkammer zur Aufnahme eines Filtermaterials aufweist, die eine ins Freie führende Gasaustrittsöffnung besitzt sowie über eine mittels eines manuell betätigbaren Ventiles verschlossene Gaseintrittsöffnung mit dem die Darmabsonderungen aufnehmenden Beutel-Innenraum verbunden ist.

Bei Personen mit einem künstlichen Darmausgang dienen sogenannte Kolostomiebeutel zur Aufnahme der Abscheidungen, d. h. insbesondere des Stuhls aus dem Darm.

Im Darm werden gleichzeitig Gase erzeugt, die dann ebenfalls in den Kolostomiebeutel gelangen und zu dessen entsprechendem Aufblähen führen. Dabei geht das Bestreben dahin, dem Patienten eine Möglichkeit zur kontrollierten Entleerung des Kolostomiebeutels von den Gasen zur Verfügung zu stellen, damit der Patient einerseits die Möglichkeit hat, die Gase zu einem Zeitpunkt abzulassen, zu dem er sich unbeobachtet fühlt, zum anderen aber auch die Möglichkeit, ein gewisses Gaspolster im Beutel zu behalten, wodurch vermieden wird, daß die Beutelwände aneinanderkleben, was ebenfalls zu Schwierigkeiten führen könnte.

Es sind bereits Einrichtungen für Kolostomiebeutel bekannt, welche eine derartige, von Hand kontrollierte Abführung der Gase gestatten. Hierzu wird beispielsweise verwiesen auf das DE-U-81 33 695. Gemäß diesem Gebrauchsmuster wird so vorgegangen, daß an dem Beutel ein Adapter vorgesehen ist, an welchen ein Schlauch angeschlossen werden kann, der zu einem getrennten, in der Tasche eines Bekleidungsstückes oder an einem Gürtel anbringbaren Ventilelement führt. Das Ventilelement umfaßt eine ein Filtermaterial aufnehmende Kammer. Diese bekannte Anordnung ist verhältnismäßig teuer und auch deswegen wenig günstig, weil in der Kleidung Löcher zu Durchführung des Schlauches bzw. Ventils angebracht werden müssen. Außerdem bestehen Bedenken bezüglich der Funktionssicherheit, da die Gefahr besteht, daß sich der Schlauch von dem am Beutel befindlichen Adapter bzw. von dem Ventil löst und zwar insbesondere dann, wenn der Schlauch in der Bekleidung des Patienten ungünstig verlegt ist oder sich der Patient besonders rasch oder übermäßig bewegt. Auf jeden Fall muß jeweils bei einem Wechsel der Bekleidung erneut das Ventil an einer geeigneten Stelle untergebracht werden, ohne daß hierbei der Schlauch von dem Kolostomiebeutel gelöst werden darf, weil ja sonst die Gefahr eines Austretens des normalerweise übelriechenden Inhaltes des Beutels besteht.

Aus der EP-A-0 064 044 ist ein Kolostomiebeutel bekannt, der eine Filterkammer zur Aufnahme eines Filtermaterials aufweist. Beim Durchströmen dieser Filterkammer erfolgt eine gewisse Beseitigung der unangenehmen Gerüche. Es ist jedoch nicht gewährleistet, daß eine vollständige Geruchsbeseitigung erreicht wird, weshalb der den Beutel benutzende Patient stets ein Austreten unangenehm riechender Gase befürchten muß. Mangelhaft bei dem bekannten Kolostomiebeutel ist weiter, daß sich in ihm kein Gaspolster aufbauen kann, da die Gase stets ungehindert ins Freie strömen können. Infolgedessen ist zu befürchten, daß die Wände des Beutels aneinander sowie an der Haut des Patienten kleben, wodurch die Aufnahmefähigkeit des Beutels vermindert werden kann und beim Ankleben an der Haut des Patienten die Gefahr besteht, daß es zu einem Wundwerden der Haut im Bereich des Beutels kommt.

Ein Beutel der eingangs erwähnten Art ist schließlich aus der US-A-2 054 535 bekannt. Bei diesem Beutel ist jedoch die Filterkammer ein getrenntes, außen am Beutel befestigtes Element, welches über einen Schlauch mit einer Austrittsöffnung des Beutels in Verbindung steht. In dieser Austrittsöffnung ist ein stöpselartiges Ventilelement vorgesehen. Bei diesem Kolostomiebeutel treten grundsätzlich die bezüglich des Beutels nach dem DE-U-81 33 695 erläuterten Mängel wegen des Vorhandenseins eines Verbindungsschlauches ebenfalls auf. Weiterhin ist dieser bekannte Beutel in der Herstellung teuer und auch bei der Benutzung wegen des gesonderten, vergleichsweise großen und steifen Filterelementes unangenehm.

Der Erfindung liegt daher die Aufgabe zugrunde, einen Kolostomiebeutel so auszubilden, daß der Patient sowohl die Möglichkeit hat, die Gase kontrolliert unter gleichzeitiger Filterung abzuführen, ohne daß die Gefahr einer ungewollten Entleerung des Beutels bestünde.

Außerdem sollen die Herstellung und Handhabung der gesamten Anordnung gegenüber den bekannten Kolostomiebeuteln wesentlich vereinfacht werden.

Zur Lösung dieser Aufgabe wird nach der Erfindung bei einem Beutel der eingangs erwähnten Art vorgeschlagen, daß die Einrichtung zur Filterung und kontrollierten Abführung der Darmgase in den Beutel integriert ist, indem in dem Beutel die Filterkammer vorgesehen ist, die mit dem restlichen Beutel-Innenraum über das Ventil in unmittelbarer Verbindung steht.

Nach der Erfindung werden also die Filter- und Entlüftungs-Einrichtung nicht getrennt vorgesehen sondern in dem Beutel integriert, wozu in dem Beutel einerseits in bekannter Weise eine besondere Kammer zur Aufnahme des Filtermaterials vorgesehen wird, jedoch andererseits zwischen Filterkammer und restlichem Beutel-Innenraum ein Ventil integriert ist. Bei Verwendung eines Beutels nach der Erfindung muß also der Patient beispielsweise nicht mehr ein besonderes Ventil unterbringen und einen hierfür geeigneten Platz in der Kleidung ausfindig machen. Da Filter und Ventil Bestandteil des Kolostomiebeutels sind, besteht auch nicht die Gefahr, daß sich Filter oder Ventil von dem Beutel lösen und dabei Beutelinhalt ungewollt austritt. Im Vergleich zu dem bekannten Kolostomiebeutel mit eingebauter Filterkammer hat der erfindungsgemäße Beutel den wesentlichen Vorteil, daß der Gasaustritt entsprechend den Bedürfnissen des Patienten beliebig gesteuert werden kann, so daß einerseits der Patient Gas nur dann ausströmen lassen kann, wenn er sich ungestört fühlt, und andererseits die Möglichkeit besteht, stets eine bestimmte Rest-Gasmenge im Beutel zu behalten, so daß die Beutelwände weder aneinander noch an der Haut des Patienten ankleben können. Beutel gemäß der Erfindung lassen sich wegen der Integration des Filterelementes und des Ventils sehr einfach und kostengünstig herstellen, so daß jedem Stoma-Patienten die Möglichkeit gegeben ist, entsprechende Beutel einzusetzen, und er nicht mehr — wie bisher meist — darauf angewiesen ist, zum Abführen der Gase den Beutel zu perforieren. Darüber hinaus ist aber auch die Handhabung im Vergleich zu den bisher üblichen, mit einer getrennten Ventilanordnung ausgerüsteten Beuteln sehr einfach.

Bei der Verwirklichung des Erfindungsvorschlages geht man zweckmäßig so vor, daß die Filterkammer durch eine nahe und etwa parallel zur oberen Beutelkante verlaufende Querwand vom Beutel-Innenraum abgetrennt ist, wobei vorteilhafterweise die Gasaustrittsöffnung am einen, die Gaseintrittsöffnung am anderen Ende der langgestreckten Filterkammer angeordnet ist. Die Querwand kann dabei extra eingesetzt oder auch nur durch eine Schweißnaht zwischen den beiden Beutelwänden gebildet sein.

Als Ventil können verständlicherweise die unterschiedlichsten Arten an sich bekannter Ventile eingesetzt werden. Eine besonders einfache Ausführung erhält man jedoch, wenn, wie nach der Erfindung vorgesehen, das Ventil ein im wesentlichen von zwei zueinander parallelen, elastisch biegbaren Plättchen gebildetes Lippenventil ist, wobei die Plättchen in der Verschlußstellung aneinander anliegen und durch seitlichen Druck in Richtung ihrer Ebene in die Öffnungsstellung auseinanderbiegbar sind. Ein derartiges Lippenventil aus zwei Plättchen läßt sich einfach, beispielsweise in einem Schweißvorgang, herstellen, was dem Bestreben nach niedrigen Herstellungskosten des Kolostomiebeutels entgegen kommt.

Günstig ist es, wenn die das Lippenventil bildenden Plättchen quer zu den Beutelwänden in einer besonderen, von dem restlichen Beutel-Innenraum mittels einer gasdurchlässigen Querwand abgetrennten Austrittskammer angeordnet sind. Das Vorhandensein einer solchen Austrittskammer trägt dazu bei, daß nach Möglichkeit nichts von dem Beutelinhalt zu dem Lippenventil kommt und dieses verstopfen könnte.

Hierzu trägt auch bei, wenn die Plättchen etwa parallel zur Querwand an einer etwa senkrecht zu dieser verlaufenden Trennwand der Austrittskammer angeordnet sind, wobei die Trennwand vorzugsweise gleichzeitig die die Gaseintrittsöffnung aufweisende Endwand der Filterkammer bildet.

Um sicherzustellen, daß das Lippenventil stets zuverlässig funktioniert, kann es günstig sein, wenn die Querwand lediglich kleine, einen Durchtritt von das Ventil blockierenden Partikeln verhindernde Durchbrechungen aufweist.

Als Filtermaterial können die unterschiedlichsten Substanzen eingesetzt werden. Einerseits ist es möglich, als Filtermaterial Adsorber-Körner, vorzugsweise Aktivkohle-Körner zu verwenden, da dann einerseits ein ausreichender Gasdurchsatz und andererseits eine entsprechend gute Adsorption erreicht werden. Es ist aber auch möglich, als Filtermaterial durch Zusätze aktivierte, bahnförmige bzw. flächenhafte Materialien, z. B. Vliese, Gewebe, Gewirke, Schäume oder aktivierte Fasern vorzusehen. Die Verwendung derartiger Filtermaterialien kann vor allem Vorteile bei deren Handhabung während der Fertigung im Vergleich zum Einsatz von Granulaten bringen. Die Aktivierung des Materials erfolgt durch entsprechende Zusätze, z. B. von Aktivkohle, Katalysatoren od. dgl. Wenn das Filtermaterial zumindest einen Katalysator umfaßt, kann eine Reinigung der Gase durch Chemiesorption erfolgen, wodurch insbesondere auch Giftstoffe entfernt werden. Der Katalysator kann als Zusatz zu einem sonstigen Filtermaterial oder als einziges Filtermaterial, ggf. in entsprechender Kombination mehrerer Katalysatoren, verwendet werden. Schließlich ist es auch noch möglich, die Filterwirkung dadurch zu verbessern, daß in den Gas-Strömungsweg vom Beutel-Innenraum zur Gasaustrittsöffnung ein von einer Membran gebildeter Filter eingeschaltet ist, um so z. B. einen ungewollten Flüssigkeitsdurchtritt zu verhindern.

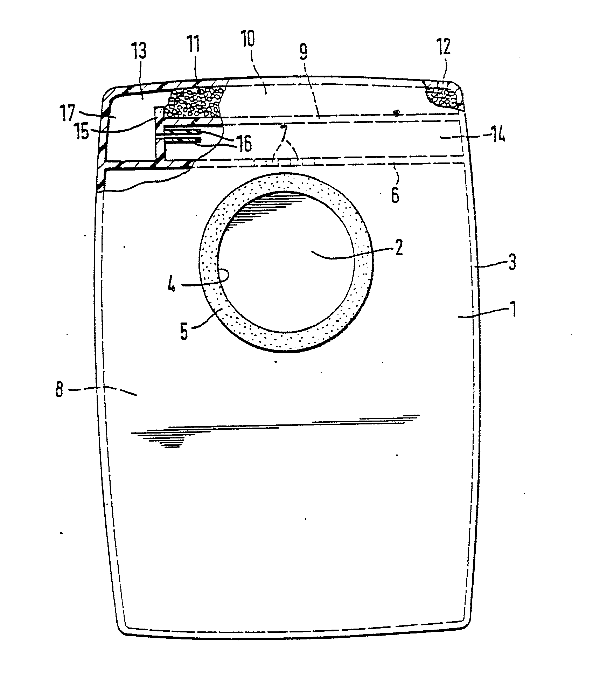

Nachstehend wird ein Ausführungsbeispiel eines erfindungsgemäß ausgebildeten Kolostomiebeutels anhand der Zeichnung näher erläutert.

Der Kolostomiebeutel besteht in bekannter Weise aus zwei zueinander parallelen Wänden 1, 2, welche entlang ihres Randes über eine Schweißnaht 3 verbunden sind. Die Wand 1 des Kolostomiebeutels weist eine Öffnung 4 auf, die von einem selbstklebenden Ring 5 umgeben ist.

Am oberen Ende des Beutels ist über eine Querwand 6 ein Beutelteil abgeteilt. Die Querwand 6 ist mit vergleichsweise kleinen Durchtrittsöffnungen 7 versehen, welche zwar einen Durchtritt von Gas aus dem Innenraum 8 des Beutels zwischen den Wänden 1, 2 unterhalb der Querwand 6 gestattet, nicht jedoch einen Durchtritt sonstiger, in dem Beutel-Innenraum 8 befindlicher Substanzen.

In dem oberen Beutelteil ist über eine Querwand 9 eine langgestreckte Filterkammer 10 abgeteilt, die mit einem entsprechenden Filtermaterial 11, beispielsweise Aktivkohle-Granulat, gefüllt ist. Die langgestreckte Filterkammer 10 weist im Bereich ihres in der Zeichnung rechten Endes oben eine Gasaustrittsöffnung 12, im Bereich des linken Endes, ebenfalls oben, eine Gaseintrittsöffnung 13 auf.

Unterhalb der Filterkammer 10 zwischen der Querwand 9 und der Querwand 6 befindet sich eine weitere, von aus dem Beutel-Innenraum 8 ausströmendem Gas durchströmte Kammer 14.

Im linken Teil der Austrittskammer 14 verläuft etwa senkrecht zu den Querwänden 6 und 9 eine Trennwand 15, welche sich von der unteren Querwand 6 nach oben bis über die gleichsam den Boden der Filterkammer 10 bildende Querwand 9 erstreckt. Diese Trennwand 15 begrenzt mit ihrer Oberkante die Gaseintrittsöffnung 13.

Die Trennwand 15 dient als Träger für zwei zueinander parallele Plättchen 16 aus elastisch biegbarem Material, die quer zu der Trennwand 15 abstehen und ein Lippenventil bilden. Dabei ist die Anordnung und Ausbildung der Plättchen 16 derart, daß sie in der Ruhestellung aneinander anliegen und durch seitlichen Druck (senkrecht auf die Zeichenebene) auseinander bewegt werden. In der Verschlußstellung, in der die Plättchen 16 federnd aneinander anliegen, kann kein Gas aus der Austrittskammer 14 über die Zwischenkammer 17 und die Gaseintrittsöffnung 13 in die Filterkammer 10 strömen. Sobald jedoch die Plättchen 16, beispielsweise in der in der Zeichnung gezeigten Weise, durch seitlichen Druck auseinander bewegt werden, kann Gas aus dem Beutel-Innenraum 8 über die Durchtrittsöffnungen 7 in der Querwand 6 in die Austrittskammer und von dort über das von den Plättchen 16 gebildete Lippenventil, die Zwischenkammer 17, die Gaseintrittsöffnung 13, die Filterkammer 10 und die Gasaustrittsöffnung 12 aus dem Beutel-Innenraum 8 ins Freie austreten. Bei Betätigung des Lippenventiles, d. h. Bewegung der Plättchen 16, wird sich im allgemeinen natürlich auch die Trennwand 15 bewegen müssen, die aus diesem Grunde aus biegbarem Material bestehen sollte.

Sobald dann der Patient den seitlichen Druck auf die Plättchen 16 einstellt, kehren diese aufgrund ihrer federelastischen Eigenschaften in die Verschlußstellung zurück, in der sie aneinander anliegen.

Die Durchtrittsöffnungen 7 in der Querwand 6 sollten so klein sein, daß sie zwar einen ungehinderten Gasdurchtritt vom Beutel-Innenraum 8 in die Austrittskammer 14 gestatten, jedoch ein Durchtreten irgendwelcher Partikel, welche sich zwischen den Plättchen 16 in der Öffnungsstellung festsetzen könnten, verhindern. Würden sich nämlich während des Gebrauchs Partikel zwischen den Plättchen 16 festsetzen, so würde das Lippenventil nach Beendigung des seitlichen Drucks auf die Plättchen 16 nicht mehr einwandfrei schließen.

Der Umstand, daß die Trennwand 15 bei dem Ausführungsbeispiel relativ weit nach oben gezogen ist, hat den Vorzug, daß unter Umständen durch die Durchtrittsöffnungen 7 und das Lippenventil aus den Plättchen 16 durchtretende Flüssigkeit sich in der Zwischenkammer 17 sammelt und nicht die Oberkante der Trennwand 15 in die Filterkammer 10 übersteigt. Auf diese Weise wird die Auslaufsicherheit des Beutels, auch wenn das Gas Flüssigkeit mitreißen sollte, besonders erhöht.

**Patentansprüche**

1. Kolostomiebeutel mit einer Einrichtung zur Filterung und kontrollierten Abführung übelriechender Darmgase, der eine besondere Filterkammer (10) zur Aufnahme eines Filtermaterials (11) aufweist, die eine ins Freie führende Gasaustrittsöffnung (12) besitzt, sowie über eine mittels eines manuell betätigbaren Ventiles (16) verschlossene Gaseintrittsöffnung (13) mit dem die Darmabsonderungen aufnehmenden Beutel-Innenraum (8) verbunden ist, dadurch gekennzeichnet, daß die Einrichtung zur Filterung und kontrollierten Abführung der Darmgase in den Beutel integriert ist, indem in dem Beutel die Filterkammer (10) vorgesehen ist, die mit dem restlichen Beutel-Innenraum (8) über das Ventil (16) in unmittelbarer Verbindung steht.

2. Kolostomiebeutel nach Anspruch 1, dadurch gekennzeichnet, daß die Filterkammer (10) durch eine nahe und etwa parallel zur oberen Beutelkante verlaufende Querwand (9) vom Beutel-Innenraum (8) abgetrennt ist.

3. Kolostomiebeutel nach Anspruch 1 und 2, dadurch gekennzeichnet, dass die Gasaustrittsöffnung (12) an einem die Gaseintrittsöffnung (13) am anderen Ende der langgestreckten Filterkammer (10) angeordnet ist.

4. Kolostomiebeutel nach einem der vorhergehenden Ansprüche, dadurch gekennzeichnet, dass das Ventil ein im wesentlichen von zwei zueinander parallelen, elastisch biegbaren Plättchen (16) gebildetes Lippenventil ist, wobei die Plättchen (16) in der Verschlussstellung aneinander anliegen und durch seitlichen Druck in Richtung ihrer Ebene in die Öffnungsstellung auseinanderbiegbar sind.

5. Kolostomiebeutel nach Anspruch 4, dadurch gekennzeichnet, dass die das Lippenventil bildenden Plättchen (16) quer zu den Beutelwänden (1, 2) in einer besonderen, von dem restlichen Beutel-Innenraum (8) mittels einer gasdurchlässigen Querwand (6) abgetrennten Austrittskammer (14) angeordnet sind.

6. Kolostomiebeutel nach Anspruch 5, dadurch gekennzeichnet, dass die Plättchen (16) etwa parallel zur Querwand (6) an einer etwa senkrecht zu dieser verlaufenden Trennwand (15) der Austrittskammer (14) angeordnet sind.

7. Kolostomiebeutel nach Anspruch 6, dadurch gekennzeichnet, dass die Trennwand (15) gleichzeitig die die Gaseintrittsöffnung (13) aufweisende Endwand der Filterkammer (10) bildet.

8. Kolostomiebeutel nach einem der Ansprüche 5 bis 7, dadurch gekennzeichnet, dass die Querwand (6) lediglich kleine, einen Durchtritt von das Ventil (16) blockierenden Partikeln verhindernde Durchbrechungen (7) aufweist.

9. Kolostomiebeutel nach einem der vorhergehenden Ansprüche, dadurch gekennzeichnet, dass als Filtermaterial Adsorber-Körner, vorzugsweise Aktivkohle-Körner, dienen.

10. Kolostomiebeutel nach einem der Ansprüche 1 bis 8, dadurch gekennzeichnet, dass als Filtermaterial durch Zusätze aktivierte, bahnförmige bzw. flächenhafte Materialien, z. B. Vliese, Gewebe, Gewirke, Schäume oder aktivierte Fasern dienen.

11. Kolostomiebeutel nach einem der vorhergehenden Ansprüche, dadurch gekennzeichnet, dass das Filtermaterial zumindest einen Katalysator umfasst.

12. Kolostomiebeutel nach einem der vorhergehenden Ansprüche, dadurch gekennzeichnet, dass in den Gas-Strömungsweg vom Beutel-Innenraum (8) zur Gasaustrittsöffnung (12) ein von einer Membran gebildeter Filter eingeschaltet ist.

## Claims

1. Colostomy bag having a device for filtration and controlled discharge of malodorous intestinal gases and having a separate filter chamber (10) which is to receive a filter material (11) and which has a gas outlet port (12) leading into the open and is connected *via* a gas inlet port (13), closed by means of a manually operable valve (16), to the interior (8) of the bag receiving the intestinal excretions, characterized in that the device for filtration and controlled discharge of the intestinal gases is integrated into the bag as a result of the filter chamber (10) being provided in the bag and being directly connected *via* the valve (16) to the remainder of the interior (8) of the bag.

2. Colostomy bag according to Claim 1, characterized in that the filter chamber (10) is separated from the interior (8) of the bag by a transverse wall extending close to and approximately parallel to the upper bag edge.

3. Colostomy bag according to Claim 1 and 2, characterized in that the gas outlet port (12) is located at one end and the gas inlet port (13) is located at the other end of the elongate filter chamber (10).

4. Colostomy bag according to one of the preceding claims, characterized in that the valve is a lip valve formed essentially by two mutually parallel, elastically flexible platelets (16), the platelets (16) being in mutual contact in the closed position and being able to be bent apart into the open position by lateral pressure in the direction of their plane.

5. Colostomy bag according to Claim 4, characterized in that the platelets (16) forming the lip valve are arranged transversely to the bag walls (1, 2) in a special outlet chamber (14) separated by means of a gas-permeable transverse wall (6) from the remainder of the interior (8) of the bag.

6. Colostomy bag according to Claim 5, characterized in that the platelets (16) are arranged approximately parallel to the transverse wall (6) on a partition (15), extending approximately perpendicular to the former, of the outlet chamber (14).

7. Colostomy bag according to Claim 6, characterized in that the partition (15) at the same time forms the end wall, containing the gas inlet port (13), of the filter chamber (10).

8. Colostomy bag according to one of Claims 5 to 7, characterized in that the transverse wall (6) has only small perforations (7) which prevent penetration of particles blocking the valve (16).

9. Colostomy bag according to one of the preceding claims, characterized in that adsorbent granules, preferably activated carbon granules, are used as the filter material.

10. Colostomy bag according to one of Claims 1 to 8, characterized in that web-shaped or two-dimensional materials activated by additives, for example nonwovens, woven fabrics, knitted fabrics, foams or activated fibres, are used as the filter material.

11. Colostomy bag according to one of the preceding claims, characterized in that a filter material comprises at least one catalyst.

12. Colostomy bag according to one of the preceding claims, characterized in that a filter formed by a membrane is inserted into the gas flow path from the interior (8) of the bag to the gas outlet port (12).

## Revendications

1. Poche à colostomie avec un dispositif de filtrage et d'évacuation contrôlée des gaz intestinaux malodorants, comportant une chambre de filtrage spéciale (10) pour loger un matériau filtrant (11) et qui comprend un orifice de sortie des gaz (12) menant à l'air libre ainsi qu'un orifice d'entrée des gaz (13) fermé par une soupape (16) actionnable manuellement, à laquelle est raccordé le compartiment intérieur de la poche (8) recueillant les sécrétions de l'intestin et caractérisée en ce que le dispositif de filtrage et d'évacuation commandée des gaz intestinaux est intégré dans la poche grâce à la présence dans celle-ci de la chambre de filtrage (10) qui est directement raccordée au reste du compartiment intérieur de la poche (8) par la soupape (16).

2. Poche à colostomie selon la revendication 1, caractérisée en ce que la chambre de filtrage (10) est séparée du compartiment intérieur de la poche (8) par une cloison transversale (9) proche du bord de la poche et à peu près parallèle à celui-ci.

3. Poche à colostomie selon les revendications 1 et 2, caractérisée en ce que l'orifice de sortie des gaz (12) est placé à une extrémité de la chambre de filtrage allongée (10) et l'orifice d'entrée des gaz (13) à l'autre extrémité.

4. Poche à colostomie selon l'une des revendications précédentes, caractérisée en ce que la soupape est une soupape à bec constituée essentiellement de deux plaquettes (16) parallèles l'une à l'autre et flexibles élastiquement, les plaquettes (16) adhérant l'une à l'autre à la position fermée et pouvant s'écarter par flexion sous l'effet d'une pression latérale dans le sens de leur plan pour se mettre en position ouverte.

5. Poche à colostomie selon la revendication 4, caractérisée en ce que les plaquettes (16) formant la soupape à bec sont disposées transversalement aux parois de la poche (1, 2) dans une chambre d'échappement (14) séparée du reste du compartiment intérieur de la poche (8) par une cloison transversale (6) perméable aux gaz.

6. Poche à colostomie selon la revendication 5, caractérisée en ce que les plaquettes (16) sont disposées à peu près parallèlement à la cloison transversale (6) contre une cloison de séparation (15) à peu près perpendiculaire à celle-ci.

7. Poche à colostomie selon la revendication 6 caractérisée en ce que la cloison de séparation (15) constitue aussi la cloison extrême de la chambre de filtrage (10) qui comporte l'orifice d'entrée des gaz (13).

8. Poche à colostomie selon l'une des revendications 5 à 7 caractérisée en ce que la cloison transversale (6) comporte uniquement de petites ouvertures (7) qui empêchent le passage de particules bouchant la soupape (16).

9. Poche à colostomie selon l'une des revendications précédentes, caractérisée en ce que ce sont des grains adsorbants, de préférence des grains de charbon actif, qui servent de matériau filtrant.

10. Poche à colostomie selon l'une des revendications 1 à 8 caractérisée en ce que ce sont des matériaux en bandes et/ou en nappes activés par des additifs, tels que des non-tissés, des tissus, des tissus à mailles, des mousses ou des fibres activées qui servent de matériau filtrant.

11. Poche à colostomie selon l'une des revendications précédentes, caractérisée en ce que le matériau filtrant comprend au moins un catalyseur.

12. Poche à colostomie selon l'une des revendications précédentes caractérisée en ce qu'un filtre formé par une membrane est inséré dans le parcours d'écoulement des gaz allant du compartiment intérieur de la poche (8) à l'orifice de sortie des gaz (12).